# EUROPÄISCHE PATENTSCHRIFT

(11) **EP 1 228 748 B1**
(45) Veröffentlichungstag und Bekanntmachung des Hinweises auf die Patenterteilung: **17.01.2007**
(21) Anmeldenummer: 01129158.0
(22) Anmeldetag: 08.12.2001
(51) Int. Cl.: A61K 8/41, A61K 8/42, A61K 8/895, A61Q 5/00, A61Q 5/02

(54) **Haarpflegemittel mit diquaternären Silikonpolymeren**
Hair conditioner comprising diquarternary silicon polymers
Agent de conditionnement de cheveux contenant des polymères de silicone diquarternaires

(30) Priorität: 31.01.2001 DE 10104033
(43) Veröffentlichungstag der Anmeldung: 07.08.2002
(73) Patentinhaber: Wella Aktiengesellschaft, 64274 Darmstadt (DE)
(72) Erfinder: Aeby, Johann, 1723 Marly (CH); Irrgang, Bernhard, 5737 Menziken (CH); Mager, Herbert, 1723 Marly (CH); Pasquier, Gilbert, 1724 Praroman (CH); Romanens, Emmanuel, 1725 Posieux (CH)

(56) Entgegenhaltungen:
- EP-A- 0 617 953
- WO-A-00/45787
- US-A- 5 306 434
- US-A- 6 136 304

## Beschreibung

Die vorliegende Erfindung betrifft ein Haarpflegemittel, mit einem Gehalt an einer Kombination eines ausgewählten quaternisierten Amins, insbesondere eines quaternisierten Amidoamins, und eines endfunktionalisertem, diquaternären Silikonpolymers.

Der Trend auf dem Haarpflegemarkt geht zu möglichst guter Pflege ("hair consciousness"). Die Anwender von Haarbehandlungsmitteln sind oft besorgt, dass ihr Haar unter den Einflüssen von Haarbehandlungen wie Dauerwelle oder Färbung oder unter Umwelteinflüssen stark leidet. Pflegeprodukte müssen helfen, insbesondere geschädigtes Haar zu pflegen, dabei das Haar aber gleichzeitig möglichst natürlich, d.h. unbelastet aussehen zu lassen. Mit herkömmlichen Haarpflegemitteln für geschädigtes Haar kann man zwar eine gute Pflege erreichen (z.B. durch Dimethicon in den Rezepturen), belastet aber auch das Haar. An kosmetischen Haarpflegemitteln werden hinsichtlich ihrer fachlichen Eigenschaften und der Umweltverträglichkeit ihrer Wirkstoffe immer höhere Anforderungen gestellt. Hierbei kommt es ganz entscheidend darauf an, dass das Mittel optimale Pflegeeigenschaften entfaltet, ohne dass unerwünschte Wirkungen beim Anwender und Belastungen der Umwelt auftreten. Auch die Glanzwirkung bei Haaren soll erhöht werden. Außerdem muß ein solches Mittel noch den Anforderungen an Stabilität genügen und leicht zu handhaben sein.

Ein bekanntermaßen hochwirksamer, kationischer Pflegestoff, insbesondere enthalten in Sprüh- und Schaumkuren, ist das Kationtensid Distearyldimethylammoniumchlorid (DSDMAC, INCI-Bezeichnung: Quaternium-18). DSDMAC wurde in der Vergangenheit in der Haarpflege breit eingesetzt und findet heute noch vor allem in Treatments, die besondere Pflegewirkungen aufweisen sollen (gute Pflege, geringe Belastung), vor allem in Verbindung mit polymeren Silikonverbindungen Verwendung. Der Nachteil dieser Substanz besteht vor allem in der schlechten biologischen Abbaubarkeit. Außerdem besitzen sie eine hohe aquatische Toxizität und belasten die Umwelt stark. Wird DSDMAC durch andere übliche quaternäre Verbindungen wie Monoalkylquats (Cetyltrimethylammoniumchlorid) oder sog. Esterquats ersetzt, werden jedoch die anwendungstechnischen Eigenschaften des DSDMAC nicht erreicht.

Andererseits kann auf kationische Tenside in Haarpflegemitteln nicht verzichtet werden. Hierbei kann das Problem auftreten, dass im Falle flüssiger kosmetischer Mittel, welche insbesondere versprüh- oder verschäumbar sein sollen, einerseits eine geringe Viskosität und andererseits eine stabile Emulsion erreicht werden müssen. Die Verwendung von beispielsweise Distearyldimethylammoniumchlorid oder analogen quaternären Ammoniumverbindungen erfüllen zwar diese Bedingungen, jedoch kommt aufgrund der genannten Nachteile die Verwendung solcher Verbindungen in kosmetischen Mitteln nicht mehr in Betracht.

Aus der WO 00/45787 ist ein Haarpflegemittel bekannt, welches dadurch gekennzeichnet ist, dass es eine Kombination enthält aus einem Amin und/oder einem quaternisierten Amin der folgenden allgemeinen Formeln: 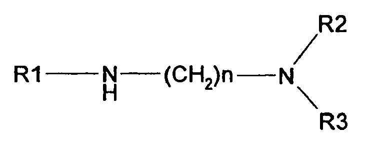 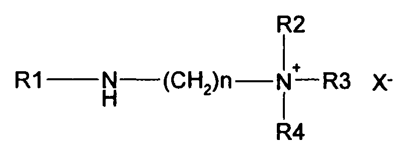 worin R1 ein Acyl- oder Alkylrest mit 8 bis 24 C-Atomen, welche verzweigt oder unverzweigt, gesättigt oder ungesättigt sein können, und wobei der Acylrest und/oder der Alkylrest mindestens eine OH-Gruppe enthalten können, R2, R3 und R4 ein Alkylrest mit 1 bis 4 C-Atomen, welcher gleich oder verschieden, gesättigt oder ungesättigt sein kann, oder H, X⁻ ein Anion, n eine ganze Zahl zwischen 1 und 10 bedeuten und mindestens einem Ester einer aliphatischen, linearen oder verzweigten Carbonsäure mit einem primären oder sekundären, verzweigten oder unverzweigten Alkohol.

EP - 617 953 und US-6 136 304 offenbaren haar Konditionierende Zusammensetzungen, die diquartenäre Silikone und nicht quarternisiertes Amidoamin enthalen.

Diese Haarpflegemittel sind aber noch nicht völlig zufriedenstellend und es bestand die Aufgabe, die Pflegeeigenschaften, insbesondere den Griff und die Kämmbarkeit des behandelten Haares sowohl im feuchten als auch im trockenen Zustand zu verbessern, ohne die Belastung zu erhöhen. Mit den nachfolgend beschriebenen, erfindungsgemäßen Haarpflegemitteln erreicht man beides: verbesserte Pflege und verringerte Belastung. Dies wird durch die unten beschriebenen Vergleichsversuche belegt.

Die gestellte Aufgabe wurde gelöst durch Bereitstellung eines Haarpflegemittels enthaltend eine Kombination aus
mindestens einem quaternisierten Amin der allgemeinen Formel

R1-NH-(CH₂)n-N⁺R2R3R4 X⁻ (Ib),

worin R1 ein Acyl- oder ein Alkylrest mit 8 bis 24 C-Atomen ist, welcher verzweigt oder unverzweigt, gesättigt oder ungesättigt sein kann, wobei der Acyl- und/oder der Alkylrest mindestens eine OH-Gruppe enthalten kann, R2, R3 und R4 unabhängig voneinander Wasserstoff, Alkyl- oder Alkoxyalkylreste mit 1 bis 6 C-Atomen sind, welche gleich oder verschieden, gesättigt oder ungesättigt und mit einer oder mehreren Hydroxygruppen substituiert sein können, X⁻ ein Anion ist und n eine ganze Zahl zwischen 1 und 10 bedeutet und (B) mindestens einem endfunktionalisierten, diquaternären Silikonpolymer.

Das erfindungsgemäße kosmetische Haarpflegemittel ist versprüh- oder verschäumbar und kann für alle bekannten kosmetischen Verwendungen, die üblicherweise die Haare betreffen, eingesetzt werden. Beispielsweise in Form einer Sprühkur, einer Schaumkur, einer Spülung oder eines Treatments. Das erfindungsgemäße Haarpflegemittel kann nach Applikation auf das trockene, feuchte oder nasse Haar entweder im Haar verbleiben oder es kann nach einer geeigneten Einwirkzeit wieder ausgespült werden. Die Einwirkzeiten hängen von der Art des Haares ab. Als allgemeine Richtlinie kann aber von Einwirkzeiten zwischen 0,5 und 30 Minuten, insbesondere zwischen 0,5 und 10 Minuten, vorzugsweise zwischen 1,0 und 5,0 Minuten ausgegangen werden.

Überaschenderweise wurde gefunden, dass eine Kombination aus einem quaternisierten Amidoamin gemäß der allgemeinen Formel (Ib) und einem endfunktionalisiertem, diquaternären Silikonpolymer in hervorragender Weise geeignet ist, kosmetische Haarpflegmittel, vorzugweise flüssige Haarpflegemittel, mit hervorragenden, verbesserten haarpflegenden Wirkungen bereitzustellen. Überraschend war auch, dass hierbei die Belastung nicht nur nicht erhöht wird (was normalerweise in Kauf genommen werden muss, wenn eine verbesserte Pflegewirkung erreicht weden will), sondern sogar verringert wird.

Bevorzugt wird ein Haarpflegemittel, worin das quaternisierte Amin gemäß allgemeiner Formel (Ib) ein quaternisiertes Amidoamin ist, worin R1 ein verzweigter oder unverzweigter, gesättigter oder ungesättigter Acylrest mit 8 bis 24 C-Atomen, welcher mindestens eine OH-Gruppe enthalten kann, bedeutet.

Bevorzugt werden für die erfindungsgemäße Kombination solche quaternisierten Amine, in denen R1 in der allgemeinen Formel (Ib) ein Fettsäurerest aus Ölen und Wachsen, insbesondere aus natürlichen Ölen und Wachsen, ist. Als Beispiele hierfür kommen Lanolin, Bienen- oder Candellilawachse in Betracht.

Bevorzugt sind auch solche quaternisierten Amine, in denen mindestens einer der Reste R2, R3 und R4 in Formel (lb) ein Rest gemäß der allgemeinen Formel CH₂CH₂OR5 bedeutet, worin R5 die Bedeutung von Alkylresten mit 1 bis 4 C-Atomen, Hydroxyethyl oder H haben kann. Die bevorzugte Größe von n in der allgemeinen Formel (lb) ist eine ganze Zahl zwischen 2 und 5.

Weiterhin bevorzugt sind quaternisierte Amine der allgemeinen Formel (lb), in denen das Anion X⁻ ein Halogenidion oder eine Verbindung der allgemeinen Formel RSO₃⁻ ist, worin R die Bedeutung von gesättigten oder ungesättigten Alkylresten mit 1 bis 4 C-Atomen hat und die Alkylreste eine oder mehrere Hydroxylgruppen enthalten können.

Als erfindungsgemäß zu verwendende quaternisierte Amine oder Aminoamine kommen beispielsweise in Betracht: REWOQUAT RTM 50 (Witco Surfactants GmbH, INCI-Bezeichnung: Ricinoleamidotrimonium methosulfate), Empigen CSC (Albright&Wilson, INCI-Bezeichnung: Cocamidopropyltrimonium Chlorid), Swanol Lanoquat DES-50 (Nikko, INCI-Bezeichnung: Quaternium-33), REWOQUAT UTM 50 (Witco Surfactants GmbH, Undecyleneamidopropyltrimonium Methosulfate).

Die erfindungsgemäßen quaternisierten Amine gemäß der allgemeinen Formel (lb) können einzeln oder in beliebigen Kombinationen miteinander verwendet werden, wobei Mengen zwischen 0,01 und 10,0 Gew.%, insbesondere zwischen 0,01 und 5,0 Gew.%, bezogen auf das fertige Produkt, eingesetzt werden können.

Geeignete diquaternäre Silikonpolymere sind ausgewählt aus Verbindungen der allgemeinen Formel (II)

R¹R²R³N⁺-A-SiR⁷R⁸-(O-SiR⁹R¹⁰)ₙ-O-SiR¹¹R¹²-A-N⁺R⁴R⁵R⁶ 2X⁻ (II)

wobei die Reste R1 bis R6 unabhängig voneinander C1-bis C22-Alkylreste bedeuten, welche Hydroxygruppen enthalten können und wobei vorzugsweise mindestens einer der Reste mindestens 8 C-Atome aufweist und die übrigen Reste 1 bis 4 C-Atome aufweisen, die Reste R7 bis R12 unabhängig voneinander gleich oder verschieden sind und C1- bis C10-Alkyl oder Phenyl bedeuten, A eine divalente organische Verbindungsgruppe bedeutet, n eine Zahl von 0 bis 200, vorzugsweise von 10 bis 120, besonders bevorzugt von 10 bis 40 ist und X⁻ ein Anion ist. Die divalente Verbindungsgruppe ist vorzugsweise eine C1- bis C12-Alkylen- oder Alkoxyalkylengruppe, die mit einer oder mehreren Hydroxylgruppen substituiert sein kann. Besonders bevorzugt ist die Gruppe -(CH₂)₃-O-CH₂-CH(OH)-CH₂-. Das Anion X⁻ kann ein Halogenidion, ein Acetat, ein organisches Carboxylat oder eine Verbindung der allgemeinen Formel RSO₃⁻ sein, worin R die Bedeutung von C1- bis C4-Alkylresten hat.

Ein bevorzugtes diquaternäres Silikonpolymer hat die allgemeine Formel

R-N⁺Me₂-A-(SiMe₂0)ₙ-SiMe₂-A-N⁺Me₂R 2 CH₃COO⁻

wobei A die Gruppe -(CH₂)₃-O-CH₂-CH(OH)-CH₂- ist, R ein Alkylrest mit mindestens 8 C-Atomen und n eine Zahl von 10 bis 120 ist.

Geeignete Silikonpolymere mit zwei endständigen, quaternären Ammoniumgruppen sind unter der INCI-Bezeichnung Quaternium-80 bekannt. Hierbei handelt es sich um Dimethylsiloxane mit zwei endständigen Trialkylammoniumgruppen. Derartige diquaternäre Polydimethylsiloxane werden von der Firma GOLDSCHMIDT unter den Handelsnamen Abil^{®} Quat 3270, 3272 und 3474 vertrieben.

Das diquaternäre Silikonpolymer kann in Mengen von 0,01 bis 10,0 Gew.%, vorzugsweise von 0,1 bis 5,0 Gew.% eingesetzt werden.

Vorteilhafterweise enthält das erfindungsgemäße Haarpflegemittel zur weiteren Verbesserung der haarkonditionierenden Eigenschaften zusätzlich mindestens einen Ester einer aliphatischen, linearen oder verzweigten Carbonsäure mit einem primären oder sekundären, verzweigten oder unverzweigten Alkohol und/oder zusätzlich mindestens ein Silikon, ausgewählt aus Alkylsiloxanen, Alkylarylsiloxanen und Aminosilikonen. Der Ester kann in einer Menge von 0,01 bis 20,0 Gew.%, vorzugsweise in einer Menge von 0,01 bis 10,0 Gew.%, insbesondere in einer Menge von 0,01 bis 5,0 Gew.% eingesetzt werden. Die Silicone und/oder Aminosilikone können als Einzelstoffe oder in Kombination miteinander in einer Gesamtkonzentration von 0,01 bis 10,0 Gew.%, vorzugsweise in einer Konzentration von 0,01 bis 8,0 Gew.% eingesetzt werden.

Die vorteilhaften Eigenschaften des Esters der besagten Carbonsäuren werden insbesondere mit Estern einer aliphatischen, linearen oder einer verzweigten C8- bis C18-Carbonsäure erzielt. Demzufolge ist ein Haarpflegemittel gemäß der vorliegenden Erfindung bevorzugt, worin der zusätzliche Ester einer aliphatischen, linearen oder verzweigten Carbonsäure 8 bis 18 C-Atome aufweist. Der mit der Carbonsäure veresterte primäre oder sekundäre, verzweigte oder unverzweigte Alkohol weist vorteilhafterweise 1 bis 6 C-Atome auf. Der für die Esterbildung primäre oder sekundäre, verzweigte oder unverzweigte Alkohol ist bevorzugt ein Monoalkohol. Ein ganz besonders bevorzugter Carbonsäureester ist Isopropylmyristat.

Geeignete Aminosilikone sind solche mit den INCI-Bezeichnungen Amodimethicone und Trimethylsilylamodimethicone. Das zusätzliche Silikon kann ein Aminosilicon der allgemeinen Formel (III) sein, 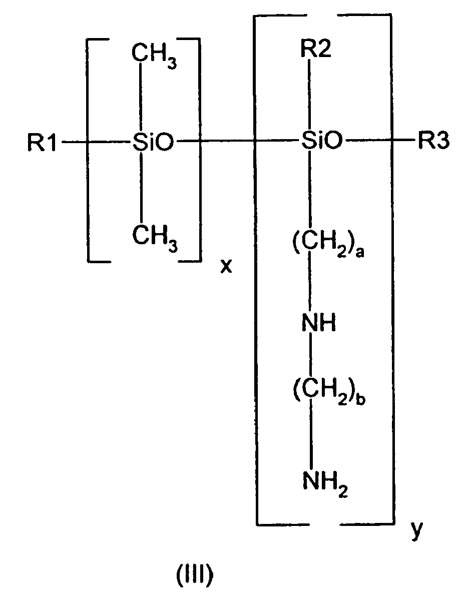 worin R1 und R2 Methyl oder OH, R3 Trimethylsilyl oder H, a eine ganze Zahl zwischen 1 und 8, b eine ganze Zahl zwischen 1 und 5, x und y ein beliebiger ganzzahliger Wert, bedeuten. Insbesondere sind aminofunktionelle Silikone gemäß allgemeiner Formel (III) geeignet, in der a eine ganze Zahl zwischen 2 und 4 und b eine ganze Zahl zwischen 1 und 3 bedeuten.

Diese aminofunktionellen Silikone haben ein Molekulargewicht zwischen 2000 und 100000 und sind beispielsweise von Dow Corning unter der Bezeichnung Silicone Emulsion No. 929, mit einem Gehalt an aminofunktionellen Silikon von ca. 35%, zu erhalten (R1, R2 = OH, R3 = H). Ebenso kann beispielsweise ein aminofunktionelles Silikon von Toshiba mit der Bezeichnung XF42-B1989 Verwendung finden. Ein solches Silikon ist erfindungsgemäß ganz besonders bevorzugt.

Ein weiteres bevorzugtes aminofunktionelles Silikon, das für das erfindungsgemäße Haarpflegemittel verwendet werden kann, ist ein Silikon gemäß Strukturformel (IV), 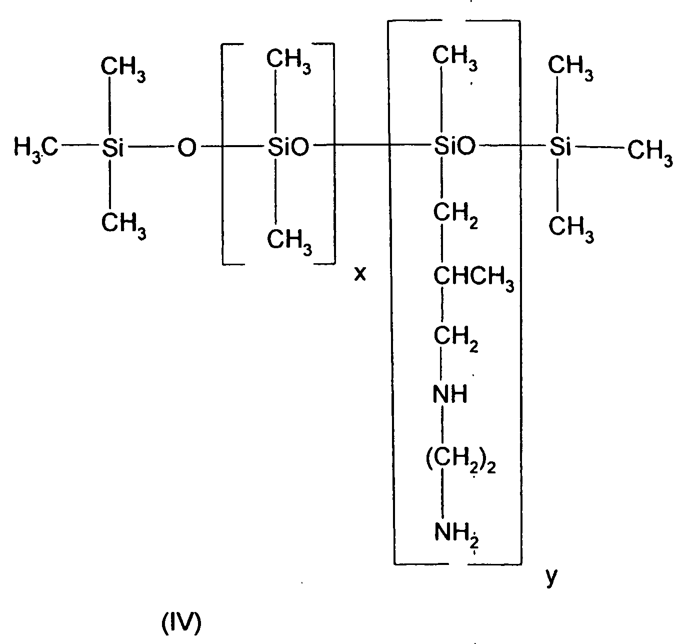 wobei das Verhältnis x zu y zwischen etwa 27.5 : 1 bis zu ungefähr 160 : 1 variieren kann. Das Molekulargewicht einer solchen Verbindung liegt dann zwischen 5000 und etwa 30000. Ein derartiges Produkt ist von Dow Corning unter der Bezeichnung Amodimethicone Q2-8075 im Handel, das ein Molekulargewicht von 8000 aufweist und ein Verhältnis von x zu y in Formel (IV) von etwa 49:1 aufweist. Dieses Amodimethicone ist in dem genannten Produkt zu etwa 37% enthalten.

Desweiteren sind in vorteilhafter Weise Silikone der allgemeinen Formel (V) als Zusatz für das erfindungsgemäße Haarpflegemittel geeignet,

(R1)₂R2Si-O-(Si(R1)₂-O)n-SiR2(R1)₂ (V)

worin R1 Methyl oder OH, R2 Methyl oder Phenyl und n eine ganze Zahl bedeuten.

Die in der allgemeinen Formel (V) genannte Verbindung soll vorzugsweise eine Viskosität von 1000 bis 1000000 cSt bei 25°C aufweisen. Besonders geeignet sind Emulsionen von Silikonen, die z.B. von der Fa. Toshiba Silicones unter der Bezeichnung XS65-B3803 vertrieben werden, wo ein Silikon gemäß allgemeiner Formel (V) mit einer Viskosität von ca. 100000 cSt eingesetzt wird (Aktivgehalt: ca 30%). Als weitere Beispiele sind zu nennen: BY 22-050A, BY22-060, BY22-062 bzw. BY22-047 von Toray Silicones.

Dem erfindungsgemäßen Haarpflegemittel kann desweiteren mindestens ein Konsistenzgeber hinzugefügt werden. Als geeignete Konsistenzgeber kommen in Betracht: Fettalkohole, insbesondere solche mit 8 bis 18 C-Atomen, vorzugsweise Cetyl- oder Cetearylalkohol, sowie Reaktionsprodukte dieser Fettalkohole mit 1 bis 10 Mol Ethylenoxid oder Propylenoxid oder ihren Mischungen, Cellulose bzw. deren Derivate wie z.B. Alkylhydroxyalkylcellulosen, beispielsweise Tylose C-Typen von Clariant, Methylhydroxyethylcellulose, beispielsweise Tylose MH-Typen von Clariant, Hydroxyethylcellulose, beispielsweise Tylose H-Typen von Clariant oder Natrosol 250-Typen von National Starch, Hydroxypropylmethylcellulose, beispielsweise Methocel-Typen von Dow Chemical, Silica bzw. sogenannte "Hydrated Silica", die beispielsweise von Degussa unter den Handelsnamen Aerosil bzw. Sipernat vertrieben werden, oder neutrale Polymere, wie beispielsweise langkettiges Polyvinylpyrrolidon (PVP). Im allgemeinen können die Konsistenzgeber in einer Konzentration von 0,1 bis 15,0 Gew.%, bevorzugt in einer Menge von 0,1 bis 10,0 Gew.%, bezogen auf die Gesamtmenge des fertigen Haarpflegemittels, eingesetzt werden.

Es hat sich herausgestellt, daß dem Haarpflegemittel weitere vorteilhafte haarpflegende Wirkung verliehen wird, wenn zusätzlich mindestens ein kationisches Tensid zugegeben wird. Die kationischen Tenside können in Mengen von 0,01 bis 5,0 Gew.%, vorzugsweise in Mengen von 0,1 bis 3,0 Gew.%, bezogen auf die Gesamtmenge des erfindungsgemäßen Haarpflegemittels, verwendet werden. Erfindungsgemäß kann für das Haarpflegemittel ein kationisches Tensid der allgemeinen Formel R¹R²R³R⁴N⁺ X⁻ verwendet werden, worin X⁻ ein beliebiges, kosmetisch verträgliches Anion, vorzugsweise Chlorid oder Methosulfat ist und die Reste R1 bis R4 gleich oder verschieden sind und verzweigte oder unverzweigte Alkylreste mit 1 bis 22 C-Atomen sind, welche mindestens eine Hydroxylgruppe enthalten können und wobei mindestens einer der Reste R1 bis R4 mindestens 8 C-Atome aufweist, oder eine Gruppe -(CH₂)n-OC(=O)-R5 ist, in der R5 ein gesättigter oder ungesättigter, verzweigter oder unverzweigter C8- bis C22-Alkylrest ist, welcher eine oder mehrere Hydroxylgruppen enthalten kann, und n eine ganze Zahl zwischen 1 und 4 ist. Vorzugsweise nicht enthalten sind Verbindungen mit R1 und R2 mit der Bedeutung von C8- bis C22-Alkylresten und mit R3 und R4 mit der Bedeutung von C1- bis C4-Alkylresten (DSDMAC-Typen).

Bevorzugte Verbindungen sind z.B. Cetyltrimethylammoniumchlorid (Arquad 16 von Akzo Nobel), oder auch sogenannte Esterquats, deren Herstellung in den europäischen Patentanmeldungen EP 0 284 036 A2, EP 0 669 391 A2 und EP 0 550 361 A1 beschrieben ist, und deren Verwendung zur Pflege von keratinischen und anderen Fasern aus der internationalen Patentanmeldung WO 96/03970 A1, und den europäischen Patentanmeldungen EP 0 636 356 A1, EP 0 689 532 A1 und EP 0 614 349 A1 bekannt ist. Die Verbindungen sind beispielsweise unter den Handelsnamen REWOQUAT WE 18, REWOQUAT WE 38 DPG (Witco Surfactants GmbH), Stepantex GS 90 (Stepan), Armocare VGH-70 (Akzo Nobel), oder Dehyquart L-80 (Henkel) erhältlich.

Weiterhin können für das erfindungsgemäße Haarpflegemittel sogenannte Betainester in üblichen Mengen eingesetzt werden, wie sie in der deutschen Patentanmeldung DE 35 27 974A1 beschrieben sind.

Selbstverständlich kann das erfindungsgemäße Haarpflegemittel alle für Haarkosmetika üblichen und bekannten Hilfs-, Wirk- und Zusatzstoffe enthalten. Grundsätzlich ist dem Fachmann bekannt, welche Hilfs- bzw. Trägerstoffe, Wirk- und Zusatzstoffe -, in der Haar- und Hautkosmetik verwendet werden, so dass die näheren Ausführungen nur beispielhaften Charakter haben und nur zur weiteren Veranschaulichung der vorliegenden Erfindung dienen sollen. Im übrigen kann hierzu auf die vorliegende Literatur verwiesen werden, die den allgemeinen Aufbau von Haarpflegemitteln beschreiben, beispielsweise SCHRADER, K., Grundlagen und Rezepturen der Kosmetika, 2. Auflage, 1989, Seiten 728 - 737 oder DOMSCH, A., Die kosmetischen Präparate, Verlag für chemische Industrie (H. Ziolkowsky, Ed.), 4. Auflage, Band 2 Seiten 212 - 230, 1992 oder JOHNSON, D. H. (Hrsg.), Hair and Hair Care, New York, 1997, Seiten 65 - 104. Die Zusatz-, Hilfs- und Trägerstoffe können in den für den Fachmann bekannten üblichen Mengen verwendet werden, insbesondere in Mengen von 0,1 bis 10,0 Gew.%.

Als übliche Hilfs-, Wirk- und Zusatzstoffe, die in dem erfindungsgemäßen Haarpflegemittel verwendet werden können, können beispielsweise genannt werden: kationische oder wasserlösliche, nicht-ionische Polymere, pflanzliche und mineralische Fette und Öle, Parfümöle, in der Haarkosmetik übliche Farbstoffe, weitere haarkonditionierende Mittel wie synthetische und natürliche Phospholipide, quaternäre Derivate der Stärke oder Cellulose, Lösungsvermittler wie beispielsweise kurzkettige primäre und sekundäre Alkohole (zum Beispiel Ethanol, 1- oder 2-Propanol), Proteine oder Proteinderivate wie beispielsweise Proteinhydrolysate (zum Beispiel Kollagen-, Keratin-, Seidenprotein- oder Weizenproteinhydrolysate), Aminosäuren (zum Beispiel Histidin, Glycin, Alanin, Serin, Threonin, Arginin, Cystein und deren Derivate, beispielsweise der Fettsäurekondensationsprodukte), Pflanzenextrakte, Vitamine oder Provitamine (zum Beispiel Biotin, Vitamin C, D-Panthenol der Derivate davon), Allantoin, Chitosan, viskositätsregulierende Stoffe wie beispielsweise Fettsäurealkanolamide, alkoxylierte Ester von Polyolen (zum Beispiel Glycerin, Sorbitol, Fructose oder Glucose, Antischuppenmittel, anorganische oder organische Säuren (zum Beispiel Essigsäure, Milchsäure, Citronensäure, Glykolsäure, Äpfelsäure, Phosphorsäure), Sonnenschutzmittel bzw. UV-Absorber, Konservierungsmittel, Antioxidantien (zum Beispiel Tocopherole oder Ester davon).

Aus der Gruppe der kationischen oder wasserlöslichen, nicht-ionischen Polymere eignen sich vor allem Polydialkyldiallylammoniumverbindungen, Polyvinylpydrrolidon, Copolymere aus Vinylpyrrolidon und Dialkylaminoalkylmethacrylat oder ein quaternisiertes Copolymere aus Vinylpyrrolidon und Dialkylaminoalkylmethacrylat, beispielsweise Dimethyl-diallylammoniumchlorid-Polymer (Merquat 100 (Calgon), Polyvinylpyrrolidon (Luviskol K 80 (BASF)), Copolymere aus Vinylpyrrolidon und Dimethylaminoethylmethacrylat (Copolymer 845, Copolymer 937, Copolymer 958 (ISP) mit Diethylsulfat quaternisierte Copolymere aus Vinylpyrrolidon und Dimethylaminoethylmethacrylat (Gafquat 734, Gafquat 755 N oder Luviquat PQ 11).

Der pH-Wert des erfindungsgemäßen Haarpflegemittels liegt vorzugsweise im neutralen bis sauren Bereich, bevorzugt zwischen pH 2,0 und 6,0. Der pH-Wert kann gegebenenfalls mit den bekannten Hilfsmitteln eingestellt werden. Hierfür eignen sich insbesondere die in kosmetischen Produkten üblichen anorganischen oder organischen Säuren, beispielsweise Phosphorsäure, Zitronensäure, Milchsäure, Äpfelsäure, Glykolsäure oder Essigsäure.

Das erfindungsgemäße Haarpflegemittel kann in den für flüssige Haarpflegemittel bekannten Applikationsformen vorliegen, insbesondere als Spülung, Sprüh- und Schaumkur oder Treatment. Das erfindungsgemäße Haarpflegemittel kann nach Aufbringen auf das Haar dort verbleiben oder ausgespült werden. Bevorzugt wird ein Haarpflegemittel, welches nach der Applikation ausgespült wird.

Das erfindungsgemäße Haarpflegemittel kann- je nach eingestellter Viskosität - vorteilhafterweise mit Hilfe üblicher Pumpen und Dosierhilfen auf das trockene oder feuchte Haar aufgebracht bzw. aufgesprüht oder verschäumt werden, beispielsweise als Aerosol. Geeignete Versprüh- oder Verschäumvorrichtung sind dem Fachmann bekannt, welche mit einem Treibmittel oder mit Hilfe mechanisch verursachter Druckerzeugung betrieben werden können. Die Auftragung erfolgt vorzugsweise im Anschluß an eine Haarwäsche.

Wenn das erfindungsgemäße Haarpflegemittel mit Hilfe eines Treibmittels appliziert, insbesondere versprüht oder verschäumt, werden soll, so kann es zusammen mit üblichen Treibmitteln in einen geeigneten Druckbehälter, insbesondere in eine Aerosoldose, in üblichen Verhältnissen oder Mengen abgefüllt werden. Als Beispiel kann hierfür ein Verhältnis von Haarpflegemittel zu Treibmittel wie 92 : 8 angegeben werden. Der Innendruck ist abhängig von dem verwendeten Behälter. Als Richtlinie kann bei einer Temperatur von 20° C von einem Druck im Bereich von 1,5 bis 8,0 bar ausgegangen werden. Als Treibmittel sind beispielsweise niedere Alkane, wie zum Beispiel n-Butan, i-Butan und Propan, Dimethylether, sowie gasförmig vorliegende Treibmittel, wie beispielsweise N₂, N₂O oder CO₂, sowie Gemische der vorstehend genannten Treibmittel, geeignet.

Unter mechanischen Versprüh- oder Verschäumvorrichtungen sind solche Vorrichtungen zu verstehen, welche das Versprühen oder Verschäumen ohne Verwendung eines Treibmittels ermöglichen. Als geeignete mechanische Versprüh- oder Verschäumvorrichtungen kann beispielsweise eine Pumpe oder ein mit einem Sprühventil versehener elastisch verformbarer Behälter, in den das erfindungsgemäße Haarpflegemittel abgefüllt wird, verwendet werden. Im Falle, daß die Abfüllung unter Druck erfolgt, kann das erfindungsgemäße Haarpflegemittel nach Öffnen des Versprüh- oder Verschäumventils kontinuierlich oder dosiert abgegeben werden. Im anderen Fall kann der zum Austritt des erfindungsgemäßen Mittels erforderliche Druck durch reversibles mechanisches Verformen des Behälters erzeugt werden.

Aufgrund der beschriebenen hervorragenden Eigenschaften der erfindungsgemäßen Kombination aus einem quaternisierten Aminoamin gemäß der vorliegenden Beschreibung mit mindestens einem endfunktionalisierten diquaternären Silikonpolymer eignet sich diese Kombination in vorteilhafterweise zur Herstellung von allgemein kosmetischen Präparationen, welche pflegende und/oder reinigende und/oder konditionierende Eigenschaften, insbesondere an Hautanhangsgebilden, entfalten sollen. Beispielsweise kann die erfindungsgemäße Kombination zur Herstellung von Mascarapräparationen, Augenbrauenpräparationen, Haarstylingmittel, Haarkonditioniermittel, Haarverformungsmitteln, Haarfärbemitteln, Haarvor- und Haarnachbehandlungsmitteln, Tonika bzw. Haarwässer oder Deodorantien verwendet werden. Insofern umfaßt die vorliegende Erfindung auch die Verwendung einer Kombination aus einem quaternisierten Amin mit mindestens einem endfunktionaliserten diquaternären Silikon gemäß vorliegender Beschreibung zur Herstellung einer kosmetischen Präparation. Auch kosmetische Präparationen enthaltend eine Kombination aus einem quaternisierten Amin mit mindestens einem endfunktionaliserten diquaternären Silikon gemäß vorliegender Beschreibung, werden daher von der vorliegenden Erfindung mitumfaßt. Das erfindungsgemäße Haarpflegemittel wird durch die nachfolgenden Beispiele näher erläutert.

### Beispiele

Die in den Tabellen angegebenen Zahlen verstehen sich als Gewichtsprozent, bezogen auf die Gesamtmenge, wenn nicht anders erläutert. Bei der Durchführung der vergleichenden Versuche wurde das Haupthaar der Probanden mit einem handelsüblichen milden Shampoo ohne konditionierende oder pflegende Wirkung wie üblich gewaschen. Dann wurde das Testprodukt auf das handtuchfeuchte Haar gegeben. Nach einer Einwirkzeit von ca. 3 Minuten wurde das Produkt wieder ausgespült. Die sensorische Beurteilung erfolgte über Halbseitentests durch in der Beurteilung von Haarqualitäten geschulte Fachleute.

**Tabelle 1: erfindungsgemäße Beispiele (A-C) und Vergleichsbeispiele (D-F):**

| | A | B | C | D | E | F |
|---|---|---|---|---|---|---|
| Cetearylalkohol | 6 | 6 | 8 | 6 | 6 | 8 |
| Paraffin | 4 | 4 | - | 4 | 4 | - |
| Lanolin | - | - | 2 | - | - | 2 |
| Lanoquat® DES-50 ⁴ | 1 | - | 1 | 1 | - | 1 |
| REWOTERIC® RTM 50 ⁵ | - | 1 | - | - | 1 | - |
| Isopropylmyristat | 4 | 2 | 2 | 4 | 2 | 2 |
| Quat. Organosilikon ¹ | 1 | 1.5 | 1 | - | - | - |
| TMS-Amodimethicon² | 0.8 | - | 1.3 | 0.8 | - | 1.3 |
| TMS-Amodimethicon-Emulsion³ | - | 1 | - | - | 1 | - |
| Cetyltrimethylammoniumchlorid | 2 | 1.3 | 1.3 | 2 | 1.3 | 1.3 |
| Dimethicone | | | | | | 1 |
| Parfüm | 0.1 | 0.1 | 0.1 | 0.1 | 0.1 | 0.1 |
| Zitronensäure | Ad pH4 | ad pH4 | ad pH4 | ad pH4 | ad pH4 | Ad pH4 |
| Wasser | Ad 100 | ad 100 | ad 100 | ad 100 | ad 100 | Ad 100 |

| | | | | | | |
|---|---|---|---|---|---|---|
| ¹: Abilquat® 3272 von Goldschmidt ²: 100%ig, Toshiba XF 49-B1989 ³: Dow Corning 939 ⁴: Quaternium-33, quaternäres Amidoammoniumsalz auf Lanolinbasis ⁵: Ricinoleamidopropyltrimonium Methosulfat | | | | | | |

**Tabelle 2: Resultate der Halbseitenversuche**

| | Griff feucht | Kämmbarkeit feucht | Griff trocken | Kämmbarkeit trocken | Belastung |
|---|---|---|---|---|---|
| A gegen D | A besser | A besser | A besser | A besser | A weniger |
| B gegen E | B besser (glatter) | B besser | B weicher (glatter) | B besser | B weniger |
| C gegen F | C besser | C besser | C besser (weicher) | C besser | C weniger |

## Patentansprüche

1. Haarpflegemittel enthaltend eine Kombination aus
(A) mindestens einem quaternisierten Amin der allgemeinen Formel
R1-NH-(CH₂)n-N⁺R2R3R4 X⁻ (Ib),
worin R1 ein Acyl- oder ein Alkylrest mit 8 bis 24 C-Atomen ist, welcher verzweigt oder unverzweigt, gesättigt oder ungesättigt sein kann, wobei der Acyl- und/oder der Alkylrest mindestens eine OH-Gruppe enthalten kann, R2, R3 und R4 unabhängig voneinander Wasserstoff, Alkyl- oder Alkoxyalkylreste mit 1 bis 6 C-Atomen sind, welche gleich oder verschieden, gesättigt oder ungesättigt und hydroxysubstituiert sein können, X⁻ ein Anion ist und n eine ganze Zahl zwischen 1 und 10 bedeutet und
(B) mindestens einem endfunktionalisierten, diquaternären Silikonpolymer.

2. Haarpflegemittel nach Anspruch 1, **dadurch gekennzeichnet, dass** das quaternisierte Amin der Formel (lb) ein quaternisiertes Amidoamin ist, worin R1 ein verzweigter oder unverzweigter, gesättigter oder ungesättigter Acylrest mit 8 bis 24 C-Atomen, welcher mindestens eine OH-Gruppe enthalten kann, bedeutet.

3. Haarpflegemittel nach einem der vorhergehenden Ansprüche, **dadurch gekennzeichnet, dass** mindestens einer der Reste R2, R3 und R4 in Formel (lb) ein Rest gemäß der allgemeinen Formel CH₂CH₂OR5 ist, worin R5 die Bedeutung von C1- bis C4-Alkylresten, Hydroxyethyl oder H haben kann.

4. Haarpflegemittel nach einem der vorhergehenden Ansprüche, **dadurch gekennzeichnet, dass** n in Formel (lb) eine Zahl zwischen 2 und 5 ist.

5. Haarpflegemittel nach einem der vorhergehenden Ansprüche, **dadurch gekennzeichnet, dass** R1 in der allgemeinen Formel (lb) ein Fettsäurerest aus Ölen und Wachsen mit 8 bis 24 C-Atomen ist ist.

6. Haarpflegemittel nach einem der vorhergehenden Ansprüche, **dadurch gekennzeichnet, dass** das Anion X⁻ ein Halogenidion oder eine Verbindung der allgemeinen Formel RSO₃⁻ ist, worin R die Bedeutung von gesättigten oder ungesättigten Alkylresten mit 1 bis 4 C-Atomen hat.

7. Haarpflegemittel nach einem der vorhergehenden Ansprüche, **dadurch gekennzeichnet, dass** das diquaternäre Silikonpolymer ausgewählt ist aus Verbindungen der allgemeinen Formel (II)
R¹R²R³N⁺-A-SiR⁷R⁸-(O-SiR⁹R¹⁰)ₙ-O-SiR¹¹R¹²-A-N⁺R⁴R⁵R⁶ 2X⁻ (II)
wobei die Reste R1 bis R6 unabhängig voneinander C1-bis C22-Alkylreste bedeuten, welche Hydroxygruppen enthalten können und wobei vorzugsweise mindestens einer der Reste mindestens 8 C-Atome aufweist und die übrigen Reste 1 bis 4 C-Atome aufweisen, die Reste R7 bis R12 unabhängig voneinander gleich oder verschieden sind und C1- bis C10-Alkyl oder Phenyl bedeuten, A eine divalente organische Verbindungsgruppe bedeutet, n eine Zahl von 0 bis 200 ist und X⁻ ein Anion ist.

8. Haarpflegemittel nach Anspruch 7, **dadurch gekennzeichnet, dass** die divalente Verbindungsgruppe A die allgemeine Formel -(CH₂)₃-O-CH₂-CH(OH)-CH₂-aufweist.

9. Haarpflegemittel nach einem der Ansprüche 7 bis 8, **dadurch gekennzeichnet, dass** das Anion X⁻ ein Halogenidion, ein Acetat, ein organisches Carboxylat oder eine Verbindung der allgemeinen Formel RSO₃⁻ ist, worin R die Bedeutung von C1- bis C4-Alkylresten hat.

10. Haarpflegemittel nach einem der vorhergehenden Ansprüche, **dadurch gekennzeichnet, dass** das diquaternäre Silikonpolymer eine Kettenlänge n von 10 - 120 Einheiten aufweist.

11. Haarpflegemittel nach einem der vorhergehenden Ansprüche, **dadurch gekennzeichnet, dass** das diquaternäre Silikonpolymer die allgemeine Formel
R-N⁺Me₂-A-(SiMe₂O)ₙ-SiMe₂-A-N⁺Me₂R 2 CH₃COO⁻
aufweist, wobei A die Gruppe -(CH₂)₃-O-CH₂-CH(OH)-CH₂- ist, R ein Alkylrest mit mindestens 8 C-Atomen und n eine Zahl von 10 bis 120 ist.

12. Haarpflegemittel nach einem der vorhergehenden Ansprüche, **dadurch gekennzeichnet, dass** es zusätzlich mindestens einen Ester einer aliphatischen, linearen oder verzweigten Carbonsäure mit einem primären oder sekundären, verzweigten oder unverzweigten Alkohol und/oder zusätzlich mindestens ein Silikon, ausgewählt aus Alkylsiloxanen, Alkylarylsiloxanen und Aminosilikonen enthält.

13. Haarpflegemittel nach einem der vorhergehenden Ansprüche, **dadurch gekennzeichnet, dass** es zusätzlich mindestens ein kationisches Tensid enthält.

14. Haarpflegemittel nach Anspruch 13, **dadurch gekennzeichnet, dass** das kationische Tensid eine Verbindung gemäß der allgemeinen Formel R¹R²R³R⁴N⁺ X⁻ ist, worin X⁻ ein Anion ist und die Reste R1 bis R4 gleich oder verschieden sind und verzweigte oder unverzweigte Alkylreste mit 1 bis 22 C-Atomen sind, welche mindestens eine Hydroxylgruppe enthalten können und wobei mindestens einer der Reste R1 bis R4 mindestens 8 C-Atome aufweist, oder eine Gruppe -(CH₂)n-OC(=O)-R5 ist, in der R5 ein gesättigter oder ungesättigter, verzweigter oder unverzweigter C8- bis C22-Alkylrest ist, welcher eine oder mehrere Hydroxylgruppen enthalten kann, und n eine ganze Zahl zwischen 1 und 4 ist.

## Claims

1. Hair care composition comprising a combination of
(A) at least one quaternized amine of the general formula
R1-NH- (CH₂)n-N⁺R2R3R4X⁻ (1b),
in which R1 is an acyl or alkyl radical having 8 to 24 carbon atoms, which may be branched or unbranched, saturated or unsaturated, where the acyl and/or the alkyl radical can contain at least one OH group, R2, R3 and R4, independently of one another, are hydrogen, alkyl or alkoxyalkyl radicals having 1 to 6 carbon atoms, which may be identical or different, saturated or unsaturated and hydroxy-substituted, X- is an anion and n is an integer between 1 and 10, and
(B) at least one end-functionalized, diquaternary silicone polymer.

2. Hair care composition according to Claim 1, **characterized in that** the quaternized amine of the formula (Ib) is a quaternized amidoamine in which R1 is a branched or unbranched, saturated or unsaturated acyl radical having 8 to 24 carbon atoms which can contain at least one OH group.

3. Hair care composition according to one of the preceding claims, **characterized in that** at least one of the radicals R2, R3 and R4 in formula (Ib) is a radical according to the general formula CH₂CH₂OR5, in which R5 can have the meaning of C1 to C4-alkyl radicals, hydroxyethyl or H.

4. Hair care composition according to one of the preceding claims, **characterized in that** n in formula (Ib) is a number between 2 and 5.

5. Hair care composition according to one of the preceding claims, **characterized in that** R1 in the general formula (Ib) is a fatty acid radical from oils and waxes having 8 to 24 carbon atoms.

6. Hair care composition according to one of the preceding claims, **characterized in that** the anion X- is a halide ion or a compound of the general formula RSO₃⁻, in which R has the meaning of saturated or unsaturated alkyl radicals having 1 to 4 carbon atoms.

7. Hair care composition according to one of the preceding claims, **characterized in that** the diquaternary silicone polymer is chosen from compounds of the general formula (II)
R¹R²R³N⁺-A-SiR⁷R⁸- (O-SiR⁹R¹⁰)ₙ-O-SiR¹¹R¹²-A-N⁺R⁴R⁵R⁶ 2X⁻ (II)
where the radicals R1 to R6, independently of one another, are C1- to C22-alkyl radicals which can contain hydroxy groups and where preferably at least one of the radicals has at least 8 carbon atoms and the other radicals have 1 to 4 carbon atoms, the radicals R7 to R12, independently of one another, are identical or different and are C1- to C10-alkyl or phenyl, A is a divalent organic compound group, n is a number from 0 to 200 and X⁻ is an anion.

8. Hair care composition according to Claim 7, **characterized in that** the divalent compound group A has the general formula - (CH₂)₃-O-CH₂-CH(OH)-CH₂-.

9. Hair care composition according to one of Claims 7 to 8, **characterized in that** the anion X⁻ is a halide ion, an acetate, an organic carboxylate or a compound of the general formula RSO₃⁻, in which R has the meaning of C1- to C4-alkyl radicals.

10. Hair care composition according to one of the preceding claims, **characterized in that** the diquaternary silicone polymer has a chain length n of 10-120 units.

11. Hair care composition according to one of the preceding claims, **characterized in that** the diquaternary silicone polymer has the general formula
R-N⁺Me₂-A- (SiMe₂O)ₙ-SiMe₂-A-N⁺Me₂R 2 CH₃COO⁻,
where A is the group - (CH₂)₃-O-CH₂-CH (OH) -CH₂-, R is an alkyl radical having at least 8 carbon atoms and n is a number from 10 to 120.

12. Hair care composition according to one of the preceding claims, **characterized in that** it additionally comprises at least one ester of an aliphatic, linear or branched carboxylic acid with a primary or secondary, branched or unbranched alcohol and/or additionally at least one silicone chosen from alkylsiloxanes, alkylarylsiloxanes and aminosilicones.

13. Hair care composition according to one of the preceding claims, **characterized in that** it additionally comprises at least one cationic surfactant.

14. Hair care composition according to Claim 13, **characterized in that** the cationic surfactant is a compound according to the general formula R¹R²R³R⁴N⁺ X⁻, in which X⁻ is an anion and the radicals R1 to R4 are identical or different and are branched or unbranched alkyl radicals having 1 to 22 carbon atoms which can contain at least one hydroxyl group and where at least one of the radicals R1 to R4 has at least 8 carbon atoms, or is a group -(CH₂)n-OC(=O)-R5, in which R5 is a saturated or unsaturated, branched or unbranched C8- to C22-alkyl radical which can contain one or more hydroxyl groups, and n is an integer between 1 and 4.

## Revendications

1. Composition de soin capillaire contenant une association de
(A) au moins une amine rendue quaternaire de formule générale
R1-NH-(CH₂)ₙ-N⁺R2R3R4 X⁻ (Ib),
dans laquelle R1 est un radical acyle ou alkyle ayant de 8 à 24 atomes de carbone, qui peut être ramifié ou non ramifié, saturé ou non saturé, le radical acyle et/ou le radical alkyle pouvant comporter au moins un groupe OH, R2, R3 et R4 représentent chacun indépendamment un atome d'hydrogène ou des radicaux alkyle ou alcoxyalkyle ayant de 1 à 6 atomes de carbone, qui peuvent être identiques ou différents, saturés ou insaturés et substitués par hydroxy, X⁻ est un anion et n représente un nombre entier compris entre 1 et 10 et
(B) au moins un polymère silicone diquaternaire fonctionnalisé en bout de chaîne.

2. Composition de soin capillaire selon la revendication 1, **caractérisée en ce que** l'amine rendue quaternaire de formule (Ib) est une amidoamine rendue quaternaire dans laquelle R1 représente un radical acyle ramifié ou non ramifié, saturé ou non saturé ayant de 8 à 24 atomes de carbone, qui peut comporter au moins un groupe OH.

3. Composition de soin capillaire selon l'une quelconque des revendications précédentes, **caractérisée en ce qu'**au moins un des radicaux R2, R3 et R4 dans la formule (Ib) est un radical de formule générale CH₂CH₂OR5, dans laquelle R5 peut avoir la signification de radicaux alkyle en C₁-C₄, hydroxyéthyle ou H.

4. Composition de soin capillaire selon l'une quelconque des revendications précédentes, **caractérisée en ce que** n dans la formule (Ib) est un nombre compris entre 2 et 5.

5. Composition de soin capillaire selon l'une quelconque des revendications précédentes, **caractérisée en ce que** R1 dans la formule générale (Ib) est un reste d'acide gras provenant d'huiles et de cires, ayant de 8 à 24 atomes de carbone.

6. Composition de soin capillaire selon l'une quelconque des revendications précédentes, **caractérisée en ce que** l'anion X⁻ est un ion halogénure ou un composé de formule générale RSO₃⁻, dans laquelle R a la signification de radicaux alkyle saturés ou insaturés ayant de 1 à 4 atomes de carbone.

7. Composition de soin capillaire selon l'une quelconque des revendications précédentes, **caractérisée en ce que** le polymère silicone diquaternaire est choisi parmi les composés de formule générale (II)
R¹R²R³N⁺-A-SiR⁷R⁸- (O-SiR⁹R¹⁰)ₙ-O-SiR¹¹R¹²-A-N⁺R⁴R⁵R⁶ 2X⁻ (II)
dans laquelle les radicaux R1 à R6 représentent indépendamment les uns des autres des radicaux alkyle en C₁-C₂₂ qui peuvent comporter des groupes hydroxy et de préférence au moins un des radicaux ayant au moins 8 atomes de carbone et les autres radicaux ayant de 1 à 4 atomes de carbone, les radicaux R7 à R12 indépendamment les uns des autres sont identiques ou différents et représentent des groupes alkyle en C₁-C₁₀ ou phényle, A représente un groupe de liaison organique divalent, n est un nombre allant de 0 à 200 et X⁻ est un anion.

8. Composition de soin capillaire selon la revendication 7, **caractérisée en ce que** le groupe de liaison divalent A correspond à la formule générale -(CH₂)₃-O-CH₂-CH(OH)-CH₂-.

9. Composition de soin capillaire selon la revendication 7 ou 8, **caractérisée en ce que** l'anion X⁻ est un ion halogénure, un acétate, un carboxylate organique ou un composé de formule générale RSO₃⁻, dans laquelle R a la signification de radicaux alkyle en C₁-C₄.

10. Composition de soin capillaire selon l'une quelconque des revendications précédentes, **caractérisée en ce que** le polymère silicone diquaternaire a une longueur de chaîne n de 10 à 120 motifs.

11. Composition de soin capillaire selon l'une quelconque des revendications précédentes, **caractérisée en ce que** le polymère silicone diquaternaire correspond à la formule générale
R-N⁺Me₂-A- (SiMe₂O)ₙ-SiMe₂-A-N⁺Me₂R 2CH₃COO⁻
dans laquelle A est le groupe -(CH₂)₃-O-CH₂-CH(OH)-CH₂-, R est un radical alkyle ayant au moins 8 atomes de carbone et n est un nombre allant de 10 à 120.

12. Composition de soin capillaire selon l'une quelconque des revendications précédentes, **caractérisée en ce qu'**elle contient en outre au moins un ester d'un acide carboxylique aliphatique, linéaire ou ramifié avec un alcool primaire ou secondaire, ramifié ou non ramifié et/ou en outre au moins un silicone, choisi parmi des alkylsiloxanes, alkylarylsiloxanes et aminosilicones.

13. Composition de soin capillaire selon l'une quelconque des revendications précédentes, **caractérisée en ce qu'**elle contient en outre au moins un tensioactif cationique.

14. Composition de soin capillaire selon la revendication 13, **caractérisée en ce que** le tensioactif cationique est un composé de formule générale R¹R²R³R⁴N⁺ X⁻, dans laquelle X⁻ est un anion et les radicaux R1 à R4 sont identiques ou différents et représentent des radicaux alkyle ramifiés ou non ramifiés ayant de 1 à 22 atomes de carbone, qui peuvent comporter au moins un groupe hydroxy et au moins l'un des radicaux R1 à R4 ayant au moins 8 atomes de carbone, ou un groupe -(CH₂)ₙ-OC(=O)-R5, dans lequel R5 est un radical alkyle en C₈-C₂₂ saturé ou insaturé, ramifié ou non ramifié, qui peut comporter un ou plusieurs groupes hydroxy, et n est un nombre entier compris entre 1 et 4.
